# EUROPEAN PATENT APPLICATION

(11) **EP 2 656 855 A2**
(43) Date of publication of application: **30.10.2013**
(21) Application number: 13171088.1
(22) Date of filing: 16.12.2009
(51) Int. Cl.: A61K 45/06, A61P 25/24, A61K 31/135, A61K 31/137, A61K 31/138, A61K 31/15, A61K 31/165, A61K 31/343, A61K 31/381, A61K 31/4406, A61K 31/445, A61K 31/45, A61K 31/4525, A61K 31/4709, A61K 31/5375, A61K 33/06, A61K 38/38

(54) **Pharmaceutical preparation for the treatment of depressive pathologies, comprising a protein with a high tryptophan-LNAAs (Large Neutral Amino Acids) ratio**

(30) Priority: 16.12.2008 IT MI20082230
(62) Divisional of application: 09179398.4
(71) Applicant: Kolfarma S.r.L., 16129 Genova (IT)
(72) Inventor: Mainardi, Paolo Francesco, 16167 Genova (IT)
(74) Representative: Ferreccio, Rinaldo

(57) **Abstract**

A pharmaceutical preparation including at least one selective serotonin re-uptake inhibitor (SSRI) or selective serotonin and noradrenaline re-uptake inhibitor (SSNRI) and a protein with a high tryptophan/LNAAs ratio, preferably α-lactalbumin, is described; the preparation may consist of a pharmaceutical composition containing both of the active ingredients or of distinct dosage units separately containing the above-stated active ingredients and is used in the treatment of depressive pathologies.

## Description

### Field of the invention

The present invention relates to the technical field of the pharmaceutical industry.

In particular, the invention refers to a pharmaceutical composition for the treatment of depressive pathologies containing a protein with a high tryptophan/LNAAs ratio.

### Background of the invention

Many neuropsychiatric disorders are associated with imbalances in the brain's production of serotonin, an important molecular mediator of cerebral neurotransmission in the paramedian limbic region of the brain, which is acknowledged to have a major action in the extrapyramidal region and to be responsible for numerous physiological and behavioural processes.

Recent findings relating to the neuropharmacology of serotonin in the central nervous system have led to the development of pharmacological agents (selective serotonin re-uptake inhibitors: SSRIs) capable of interacting with the serotoninergic system. By blocking cellular re-uptake, such drugs increase the availability of serotonin in the synapses.

Serotonin is involved in the neuropsychiatric disorders typical of Parkinson's disease, Huntington's disease and in some forms of cerebrovascular diseases. SSRIs are used in these pathologies to treat depression, anxiety, dopamine-mimetic psychosis, emotional instability, obsessive-compulsive disorders and a suicidal tendency. SSRIs have also proven effective in the treatment of emotionality subsequent to cerebral infarction.

Today, there are various SSRIs in existence, some of which also act on the re-uptake of noradrenaline and are known by the name SSNRI. Such drugs saw great success in the 1990s, to such an extent that they were dubbed the "happy pill".

Recently, some English researchers (Porter R.J. et al., Psychopharmacology (2003) 165:216-221) have reassessed all the results of the studies carried out by the FDA for licensing new SSRIs from 1989 to 1998, and concluded that these drugs are of doubtful efficacy, especially in more serious cases of depression. The study reports that the efficacy of SSRIs in moderate depression is 50% relative to placebo. This means that identical levels of clinical improvement were obtained in those patients to whom the drug was administered and in those to whom the placebo was administered. In severe cases of depression, SSRIs and SSNRIs appear to produce a greater antidepressive action relative to placebo, but the analysis by these researchers reveals that it is the placebo which would appear to lose efficacy.

Data from the scientific literature indicate a low plasma level of tryptophan in depressed patients and a low tryptophan/LNAAs (LNAAs = Large Neutral Amino Acids) ratio, which results in a low cerebral uptake of tryptophan with consequently reduced synthesis of serotonin in the brain (Harry T Chugani, MD and Diane C Chugani, PhD, Imaging of Serotonin Mechanisms in Epilepsy, Epilepsy Curr. 2005 November; 5(6): 201-206).

Serotonin is in fact synthesised in the brain solely from tryptophan, an essential amino acid which is obtained by dietary intake. Tryptophan competes with the other neutral amino acids (Large Neutral Amino Acids: LNAAs), which are tyrosine, valine, methionine, isoleucine, leucine and phenylalanine, for passage through the blood-brain barrier and tryptophan's ability to enter the central nervous system will thus depend on the ratio between its concentration and that of the other competing LNAAs.

Another study in the literature shows that the efficacy of SSRIs depends on the basal level of plasma tryptophan. This result may be interpreted in the light of the fact that a low level of plasma tryptophan, or more specifically of the tryptophan/LNAAs ratio, corresponds to low cerebral uptake of tryptophan and consequently to a low level of serotonin synthesis, and a low tryptophan/LNAAs ratio corresponds to a low level of serotonin in the brain. If little serotonin is present in the brain, increasing its period of action by inhibiting re-uptake is not sufficient to produce a good clinical result.

Administering tryptophan or 5-hydroxytryptophan has not provided clear clinical results in major depression. This lack of efficacy may be correlated with a low level of intestinal absorption of the free amino acids. This is because a free amino acid, obtained by dietary intake, is not readily assimilated by the digestive tract because it is not recognised as a foodstuff and is thus eliminated.

Furthermore, the process of assimilating free amino acids through the intestinal membrane involves the same competition between the LNAAs and free tryptophan will thus be absorbed as a function of the concentration of the other LNAAs.

The only reported effect is that supplementation with tryptophan or 5-hydroxytryptophan makes it possible to reduce the times within which the SSRIs or SSNRIs exhibit their efficacy, which usually amount to about one month. Previous studies by the Applicant, which were the subject matter of Italian patent application GE2006A000013, led to the identification of a protein, α-lactalbumin, which is readily assimilated by the body and is characterised by a high content of tryptophan and a low content of the other LNAAs.

The above-stated application suggested using α-lactalbumin in the production of a medicament for the treatment of neuropsychiatric diseases, such as Parkinson's disease, depressive pathologies, epilepsy and the like.

### Brief description of the invention

The problem underlying the present invention was to provide a novel pharmaceutical preparation for the treatment of depressive pathologies.

Such a problem has been solved by a pharmaceutical preparation including at least one selective serotonin re-uptake inhibitor (SSRI) or selective serotonin and noradrenaline re-uptake inhibitor (SSNRI) and a protein with a high tryptophan/LNAAs ratio.

Preferably, α-lactalbumin is used as the protein with a high tryptophan/LNAAs ratio.

According to one aspect of the present invention, the pharmaceutical preparation comprises at least one first dosage unit including at least one selective serotonin re-uptake inhibitor (SSRI) or selective serotonin and noradrenaline re-uptake inhibitor (SSNRI) and a pharmaceutically acceptable carrier and at least one second dosage unit including a protein with a high tryptophan/LNAAs ratio, preferably α-lactalbumin, and a pharmaceutically acceptable carrier, wherein said first and second dosage units are distinct units intended for simultaneous or separate administration.

According to another aspect of the present invention, the pharmaceutical preparation consists of a pharmaceutical composition comprising at least one selective serotonin re-uptake inhibitor (SSRI) or selective serotonin and noradrenaline re-uptake inhibitor (SSNRI) and a protein with a high tryptophan/LNAAs ratio, preferably α-lactalbumin, together with a pharmaceutically acceptable carrier.

Preferably, said SSRI is selected among the group comprising cericlamine, citalopram, clovoxamine, cyanodothiepin, dapoxetine, escitalopram, femoxetine, 4-(2-fluorophenyl)-6-methyl-2-(1-piperazinyl)-thieno[2,3-D]pyrimidine, fluoxetine, fluvoxamine, ifoxetine, indalpine, indeloxazine, litoxetine, paroxetine, sertraline, viqualine and zimeldine, and said SSNRI is selected among the group comprising venlafaxine, duloxetine, milnacipran and desvenlafaxine.

The protein with a high tryptophan/LNAAs ratio, preferably α-lactalbumin, is contained in each dosage unit in an amount ranging from 0.1 to 2.0 g, preferably between 0.3 and 1.0 g, while the SSRI or SSNRI is contained in each dosage unit or in the composition in the amounts normally provided for their clinical use.

α-Lactalbumin of bovine origin is preferably used as the protein with a high tryptophan/LNAAs ratio, not least by virtue of the fact that it has a greater content of tryptophan in comparison with other commercially available α-lactalbumins. α-Lactalbumin produced by various manufacturers, such as for example Davisco Food International (USA) and Borculo Domo (NL), is commercially available.

The α-lactalbumin is generally obtained from cow's milk whey by purification by means of separative chromatography, although flash chromatography methods may also be used.

The composition or the dosage units according to the invention may be administered by various routes, although oral administration is preferred.

Dosage forms for oral administration may include syrups, sachets of soluble powder or granules, tablets, capsules, film coated tablets and the like.

In a further aspect, the invention relates to a kit for the treatment of depressive pathologies, comprising at least one first dosage unit, containing at least one selective serotonin re-uptake inhibitor (SSRI) or at least one selective serotonin and noradrenaline re-uptake inhibitor (SSNRI) together with a pharmaceutically acceptable carrier, and at least one second dosage unit containing a protein with a high tryptophan/LNAAs ratio, preferably α-lactalbumin, together with a pharmaceutically acceptable carrier, as well as instructions for the concomitant use of said at least one first and at least one second dosage units in the treatment of depressive pathologies.

Finally, the invention refers to α-lactalbumin for use in the treatment of depressive pathologies in association with at least one selective serotonin re-uptake inhibitor (SSRI) or selective serotonin and noradrenaline re-uptake inhibitor (SSNRI).

It has been found experimentally that administering proteins with a high tryptophan/LNAAs ratio, in particular α-lactalbumin, is the best way of increasing the plasma level of tryptophan, thanks to the high bioavailability of α-lactalbumin, and thus enables greater efficacy of SSRI or SSNRI antidepressant drugs.

Administering α-lactalbumin in the course of treatment with SSRIs or SSNRIs achieves a synergistic action between the increased level of serotonin in the brain and its increased period of action, specifically thanks to the high bioavailability of α-lactalbumin.

The protein with a high tryptophan/LNAAs ratio, in particular α-lactalbumin, may be administered simultaneously with the SSRI or SSNRI, for example by using a pharmaceutical form which contains both of the active ingredients or by simultaneously administering two pharmaceutical forms of which one respectively contains the protein with a high tryptophan/LNAAs ratio and the other the SSRI or SSNRI. The two active ingredients may also be administered at different times over the course of the day on the basis of a dosage regimen specified by a doctor.

The antidepressant action of the SSRI or SSNRI taken together with the protein with a high tryptophan/LNAAs ratio is appreciably greater than the action exerted by the individual components.

Magnesium and group B vitamins may also be added to the preparation to promote the conversion of tryptophan into serotonin.

The present invention will be further described with reference to some examples which are provided by way of non-limiting illustration.

### Example 1

| | |
|---|---|
| Purified bovine α-lactalbumin* | 500 mg |
| Fructose | 230 mg |
| Fluoxetine hydrochloride | 22.1 mg** |

| | |
|---|---|
| * produced by Davisco Food International (USA) ** corresponding to 20 mg fluoxetine base. | |

The above-listed ingredients in powder form were mixed until homogeneous and hard gelatin capsules were filled with the resultant mixture using conventional pharmaceutical manufacturing methods.

### Example 2

**α-Lactalbumin tablets:**

| | |
|---|---|
| Purified bovine α-lactalbumin* | 500 mg |
| Fructose | 200 mg |
| Microcrystalline cellulose | 30 mg |
| Magnesium stearate | 10 mg |
| Flavouring agent | 10 mg |

| | |
|---|---|
| * produced by Davisco Food International (USA). | |

The above-listed ingredients in powder form were mixed until homogeneous and tablets were produced with the resultant mixture using conventional pharmaceutical manufacturing methods.

**Fluoxetine tablets**

| | |
|---|---|
| Fluoxetine hydrochloride | 22.1 mg |
| Lactose | 50.0 mg |
| Maize starch | 3.0 mg |
| Water (per 1000 tablets) | 30.0 ml* |
| Maize starch | 20.0 mg |
| Magnesium stearate | 0.5 mg |

| | |
|---|---|
| * The water evaporates during processing. | |

The fluoxetine hydrochloride was mixed with the lactose until homogeneous. The minor amount of maize starch was mixed with the water and the resultant paste was added to the mixture of fluoxetine and lactose until a uniform moist mass was formed. The remaining maize starch was then added to the moist mass and mixing was continued until uniform granules were obtained. The latter were ground and screened through a ¼ inch screen and oven-dried to the desired moisture content. The dried granules then ground using a ¼ mesh screen. The magnesium stearate was then mixed with the granules and tablets were obtained from the resultant mixture using a conventional apparatus.

The α-lactalbumin tablets and the fluoxetine tablets were packaged separately in respective blister strips. The blister strips of α-lactalbumin tablets and the blister strips of fluoxetine tablets were in turn packaged in a paperboard box, into which was also introduced an information leaflet describing therapeutic indications, methods of taking and dosage.

The effects of the pharmaceutical preparation according to the present invention may be verified by using the animal models commonly used for evaluating antidepressant agents, such as for example the forced swim test on rats according to Cristiano M.S. et al. ("Neonatal treatment with fluoxetine reduces depression behaviour induced by forced swim in adult rats." Arq Neuropsiquiatr 2002; 60 (4): 928-932) or the forced swim test on mice according to Takahiro N. et al. ("Antidepressant-like effect of apigenin and 2,4,5-trimethoxycinnamic acid from Perilla frutescens in the forced swimming test" Biol. Pharm. Bull. 2002; 26(4): 474-480) or finally the murine model of chronic stress according to D'Aquila P.S. et al. ("Effects of chronic mild stress on performance in behavioral tests relevant to anxiety and depression". Physiol. Behav. 1994; 56 (5): 861-867).

## Claims

1. A pharmaceutical preparation including at least one selective serotonin re-uptake inhibitor (SSRI) or at least one selective serotonin and noradrenaline re-uptake inhibitor (SSNRI), and α-lactalbumin, wherein α-lactalbumin is in an amount ranging from 0.1 to 2.0 g.

2. A pharmaceutical preparation according to claim 1, wherein said at least one selective serotonin re-uptake inhibitor (SSRI) or at least one selective serotonin and noradrenaline re-uptake inhibitor (SSNRI) is contained in at least one first dosage unit together with a pharmaceutically acceptable carrier and said α-lactalbumin is contained in at least one second dosage unit together with a pharmaceutically acceptable carrier, said dosage units being distinct units intended for simultaneous or separate administration.

3. A pharmaceutical preparation according to claim 1, consisting of a pharmaceutical composition comprising said at least one selective serotonin re-uptake inhibitor (SSRI) or selective serotonin and noradrenaline re-uptake inhibitor (SSNRI) and said α-lactalbumin together with a pharmaceutically acceptable carrier.

4. A pharmaceutical preparation according to any one of the preceding claims, wherein said SSRI is selected among the group comprising cericlamine, citalopram, clovoxamine, cyanodothiepin, dapoxetine, escitalopram, femoxetine, 4-(2-fluorophenyl)-6-methyl-2-(1-piperazinyl)-thieno[2,3-D]pyrimidine, fluoxetine, fluvoxamine, ifoxetine, indalpine, indeloxazine, litoxetine, paroxetine, sertraline, viqualine and zimeldine, and said SSNRI is selected among the group comprising venlafaxine, duloxetine, milnacipran and desvenlafaxine.

5. A preparation according to claim 2, wherein α-lactalbumin is contained in an amount ranging from 0.3 to 1.0 g.

6. A preparation according to claim 3, wherein α-lactalbumin is contained in an amount ranging from 0.3 to 1.0 g.

7. A pharmaceutical preparation according to claim 2 or 5, wherein said at least one second dosage unit further contains magnesium and/or group B vitamins.

8. A pharmaceutical preparation according to claim 3 or 6, wherein said pharmaceutical composition further includes magnesium and/or group B vitamins.

9. A pharmaceutical preparation according to any one of the preceding claims, **characterized in that** it is suitable for oral administration.

10. A pharmaceutical preparation according to claim 3, 6 or 8, wherein said pharmaceutical composition is in the form of tablets, syrups, capsules, film coated tablets or sachets of powder or granules.

11. A pharmaceutical preparation according to claim 2, 5 or 7, wherein said at least one first and at least one second dosage units are independently in the form of tablets, syrups, capsules, film coated tablets or sachets of powder or granules.

12. A kit for the treatment of depressive pathologies, comprising at least one first dosage unit, containing at least one selective serotonin re-uptake inhibitor (SSRI) or at least one selective serotonin and noradrenaline re-uptake inhibitor (SSNRI) together with a pharmaceutically acceptable carrier, and at least one second dosage unit containing α-lactalbumin in an amount ranging from 0.1 to 2.0 g, together with a pharmaceutically acceptable carrier, as well as instructions for the concomitant use of said at least one first and at least one second dosage units in the treatment of depressive pathologies.
